# EUROPEAN PATENT SPECIFICATION

(11) **EP 4 390 395 B1**
(45) Date of publication and mention of the grant of the patent: **16.07.2025**
(21) Application number: 22216500.3
(22) Date of filing: 23.12.2022
(51) Int. Cl.: G01N 33/50, G01N 33/68, G01N 1/34

(54) **SAMPLE PREPARATION METHOD FOR ANALYSIS OF THE SERUM PEPTIDOME BY TANDEM MASS SPECTROMETRY**
PROBENVORBEREITUNGSVERFAHREN ZUR ANALYSE DES SERUMPEPTIDOMS DURCH TANDEM-MASSENSPEKTROMETRIE
PROCÉDÉ DE PRÉPARATION D'ÉCHANTILLON POUR L'ANALYSE DU PEPTIDOME SÉRIQUE PAR SPECTROMÉTRIE DE MASSE EN TANDEM

(43) Date of publication of application: 26.06.2024
(73) Proprietor: Uniwersytet Gdanski, 80809 Gdansk (PL)
(72) Inventor: Kote, Sachin, 80-116 Gdansk (PL); Marek-Trzonkowska, Natalia, 80-752 Gdansk (PL); Czaplewska, Paulina, 80-461 Gdansk (PL); Müller, Marc, 80-462 Poland (PL); Pirog, Artur, 80-312 Gdansk (PL); Faktor, Jakub, 80-170 Gdansk (PL)
(74) Representative: Czarnik, Maciej

(56) References cited:
- WO-A1-2011/062270
- TIAN RUIJUN ET AL: "Selective extraction of peptides in acidic human plasma by porous silica nanoparticles for peptidome analysis with 2-D LC-MS/MS", JOURNAL OF SEPARATION SCIENCE, vol. 30, no. 14, 1 September 2007 (2007-09-01), DE, pages 2204 - 2209, XP093044378, ISSN: 1615-9306, DOI: 10.1002/jssc.200700156
- JURAJ LENCO ET AL: "MS/MS library facilitated MRM quantification of native peptides prepared by denaturing ultrafiltration", PROTEOME SCIENCE, BIOMED CENTRAL, LONDON, GB, vol. 10, no. 1, 4 February 2012 (2012-02-04), pages 7, XP021123765, ISSN: 1477-5956, DOI: 10.1186/1477-5956-10-7
- TARA K SIGDEL ET AL: "Optimization for peptide sample preparation for urine peptidomics", CLINICAL PROTEOMICS, BIOMED CENTRAL LTD, LONDON, UK, vol. 11, no. 1, 25 February 2014 (2014-02-25), pages 7, XP021178720, ISSN: 1559-0275, DOI: 10.1186/1559-0275-11-7
- YU JING ET AL: "Insights into the structure-performance relationships of extraction materials in sample preparation for chromatography", JOURNAL OF CHROMATOGRAPHY A, ELSEVIER, AMSTERDAM, NL, vol. 1637, 16 December 2020 (2020-12-16), XP086453236, ISSN: 0021-9673, [retrieved on 20201216], DOI: 10.1016/J.CHROMA.2020.461822

## Description

The invention refers to a qualitative and quantitative method for serum peptidome profiling for human samples. The invention approach is simple, cost-effective, fast, and comprehensive for serum peptidomics profiling. These serum peptides can be used for biomarker discovery, diagnosis, prognosis, prediction, monitoring, and differentiation of various human disease types.

### State of the art

Peptidomics targets and analyses the peptides (their length, distribution, protein origin, or de-novo sequencing). Peptides are protein fragments with various functions. As they regulate multiple physiological and pathological processes, alterations in peptidome may reflect both beneficial or deleterious phenomena. Thus, peptidome analysis in body fluids such as serum samples is a promising approach for identifying peptide biomarkers of various conditions and monitoring patient response to the applied treatment. The peptidome [1] is considered as low molecular-weight peptides (less than 15 kDa). Endogenous peptides primarily act as a messenger (cytokines, hormones, growth factors, etc.) and indicate various biological processes. Hence, these peptides can reflect the health or disease state of the person.
In addition, the endogenous peptides also provide insight into proteolytic activity, degradation, and degeneration and allow the study of disease microenvironments.

Even though peptidomics has been introduced since the decade, the serum peptidome analysis has consistently been restricted for various reasons; a) enormous complexity because serum contain elements of all proteins produced in the body, b) physiologically status of high abundant proteins such as serum albumin, immunoglobulins are almost 90% of total serum protein by weight, hence these abundant proteins mask the detection of other proteins, especially low abundance proteins or peptides, c) simultaneously limit detection of low abundant peptides with diagnostic potential, d) unequal concentrations of low and high abundant proteins (around 95% of high abundant proteins and less than 5% presence of low abundant) in the total serum protein concentrations, e) instability of low abundant proteins or peptides (hormones or small secreted proteins or possible diagnostic or prognostic biomarkers), f) depletion steps, fractionation and precipitation methods for the removal of high abundant proteins are overwhelming that compromise the number, yield of diagnostic peptides identified and important biological information can be lost in the background noise. Hence, all previously described methods for serum peptide analysis were characterized by low numbers of identified peptides, and no quantitative methods for serum peptidome analysis were introduced.

WO 2011/062270 A1 discloses a sample preparation method for peptides from serum to be used in a liquid chromatography tandem mass spectrometry (LC-MS/MS) application; using acidic buffer to dissociate peptides from blood proteins like albumin, protection from acidic proteases by mesoporous particles (PG1); detection by densitometry of around 10,000 total peptides with 1.8 nm pore particles and glycine buffer; pH 2.5 citrate-phosphate buffer resulting in more release of peptides around 10 kDa compared to glycine buffer.

The invention includes the unique sample preparation steps for serum peptidomics qualitative and quantitative analysis as defined in the claims.

Using serum for early screening and diagnosis is vital to develop an effective therapy for many diseases. Data from previous literature showed that the serum peptidomics approach had been used for the diagnosis of various range of diseases such as lung cancer study included 64 patients, identified only 38 potential peptide biomarkers to distinguish lung cancer patients and healthy controls [2], reproductive disorders, study included 24 patients after statistical analysis, 23 peaks/peptides showed significantly different peak intensities in the pregnancy-induced hypertension (PIH) patients compared with the controls at an average [3], Gestational Diabetes Mellitus study included 200 patients total of 297 identified peptides, were significantly differentially expressed in the gestational diabetes mellitus (GDM) group compared with control [4], (did not perform any quantification) Rheumatoid Arthritis study included 35 patients, trial showed 113 peaks that discriminated rheumatoid arthritis (RA) from OA (primary osteoarthritis) and 101 peaks that discriminated RA from healthy controls (HC) [5], ulcerative colitis study included 78 patients 732 peptides were successfully identified by this method [6], etc. However, the ultimate success of these previously published methods is severely limited due to the low number of identified markers. There is a need to develop a simple and effective serum sample preparation method that should allow the quantification of serum peptidomics data. There are known methods for low identification number of peptides or quantification of few peptides (less than 20 peptides). Complex sample preparation that includes depletion of high abundant proteins step, precipitation step, lysis, protein labeling by fluorescence dye, multistep peptide purification and desalting step, require additional set up of chromatography, etc. Whereas in our approach we bypass all these time and cost-consuming steps.

In the past, several patents were published with methods, devices, and kits aiming to tackle biomarker discovery with different approaches such as protein/peptide levels and immunoassay [7] [8] [9] [10] [11], plasma enzyme levels [12], etc. Some patent [11] reported one or more peptide biomarkers for diagnosing cardiovascular disease (CVD) by mass spectrometry (MS) analysis such as Matrix-Assisted Laser Desorption/Ionization (MALDI), [11] Time-of-Flight (TOF). Protein quantification data was missing [8] [9] in these report. Samples from [11] total 20 patients serum samples were analyzed for free and bound peptides on total serum proteins. Moreover, considering a single biomarker such as glycogen phosphorylase- BB (GPBB) [7] or protein alpha- 1 antitrypsin [13], L-gultamine hydroxylamine glutamyl transferase (L-GHGT) and gamma glutamyl hydroxamate synthetase (GGHS) activity [12] for stroke might be too risky to rely on accurate diagnosis. On the contrary, our approach is high throughput and comprehensive, identifying 15000-16000 and successfully quantifying 1900-2100 serum peptides from 15 patients.

The invention differs from those available approaches; it created a serum peptidomics (based on amino acid sequences) approach. It is purely based on sequencing natural peptides of amino sequences; however, there are significant differences concerning existing methods such as in samples preparation, time, and cost-wise with the proteomics/peptidomics approach [9] [10] [11] for diagnostic and prognostic cardiovascular disease. Our novel peptidomics approach is unique in many stages;
1. Major difference is focusing on serum peptides and not proteins;
2. The unique step is to enrichment of peptides in the analyzed serum sample by incubation of the sample with a citrate-phosphate buffer with pH 3.1-3.6 in water with a purity ≥ 99.9%, wherein the buffer and serum volume ratio is 29:1 and the buffer and serum are mixed at a temperature of range 4 to 8°C to dissociate peptides from highly concentrated serum proteins; removing of proteins equal or bigger than 15 kDa from peptide enriched and simultaneously purification of peptides using hydrophilic-lipophilic balanced columns to obtain peptides smaller than 15 kDa. Therefore, it is characterized by the unexpected use of low pH buffer for the enrichment of peptides from complexes with high abundant/carrier proteins;
3. In the past, they used the depletion step, [9] [14] [6] removal serum high abundant proteins by precipitation method. It is more evident that depleting these high-abundance proteins may exclude the unique peptide biomarkers resulting in a poor prognosis/diagnosis [15] with existing biomarkers. Therefore, an approach was developed without depletion and precipitation steps, which allows us to yield more peptides and isolate possible carrier peptides from highly abundant proteins;
4. The invention does not need to use a clean-up kit such as Ettan 2D clean or any quantification kit proteins [8] [9]; all these steps are time and cost-consuming;
5. For the high sensitivity, past patents [9] used fluorescent dye (Cy3, Cy5) for proteins labeling. Reading and imaging of proteome profile from the dye-based gel needs a specific instrument (The Typhoon instrument, GE Healthcare needed for variable-mode imager that produces digital images of radioactive, fluorescent, or chemiluminescent samples), which again needs investment for time and money. Therefore, it is a complex sample preparation procedure and requires an experienced person to perform this analysis;
6. The sixth and most crucial difference is the protein enzymatically digestion step. To date, any proteomics analysis is impossible to perform without enzymatic digestion (trypsin, chymotrypsin, c-lysine, etc.). On the contrary, the approach is completely free from additional manipulation steps such as reduction, alkylation, and enzymatic (tryptic) digestion steps. It also allows to identify natural/endogenous peptides (which can serve as unique biomarkers) without any modifications;
7. The invention does not require any lysis buffer for isolating and desaturating the proteins or peptides of interest and keeping them in a stable environment;
8.Most of the reports show that they are focused on only the identification of proteins/peptides, however, the invention is based on performance on both (qualitative and quantitative) analysis;
9. The instrumentation (we used the most advanced mass spectrometry model, Exploris 480 MS), previously, for the separation and identification of peptides, the inventors used two dimensional difference gel electrophoresis (2D DIGE), Surface-enhanced laser desorption/ionization (SELDI) or modified liquid chromatography-Matrix-Assisted Laser Desorption/lonization mass spectrometry (LC-MALDI), Matrix-Assisted Laser Desorption/lonization-Time Of Flight mass spectrometry MALDI TOF/MS based platforms for various biological samples. These platforms are less sensitive and comprehensive than orbitrap-based mass spectrometer. In short, the invention method been developed as a novel, without precipitation, depletion, lysis, protein labeling by fluorescent dye, gel electrophoresis, desalting etc., and a direct qualitative, quantitative, comprehensive serum peptidomics approach;
10. Due to simplicity and fewer steps to perform during sample preparation, it facilitates working high throughput sample preparation (100-200 samples/day);
11. Serum is the most commonly available sample in the world and routinely used for pathological tests so our approach is globally applicable for all serum samples (human and non-human serum samples such as canines, cats, swine, horses, cow, cheetah, leopard, etc.);
12. This Multi-biomarkers peptides panel possible easily translations for the various purpose early diagnosis, prognosis, monitoring and predictions of health issues. Moreover, can be used for development of various Point Of Care Diagnostic (POCD) platforms for various disease;
13. Method is easily tunable; deepening on interest, we can enrich short (less than 3 kilo Dalton/kDa) and long (less than 10 kilo Dalton/kDa) serum peptide length distribution from clinical samples,
14.The impact of our innovation also has tremendous potential to be translational, commercialization, and direct finding to serum peptide biomarkers.

The invention aimed to develop a sample preparation and comprehensive method for qualitative, quantitative analysis of serum peptidome samples. Moreover, this novel approach can be used routinely to discover multi-biomarkers for diagnosing, prognosis, monitoring, and predicting various diseases. This approach facilitates to performed of both qualitative and quantitative analysis of serum peptidomics, mainly enables to DDA (data-dependent acquisition) to identify a total of 15000-17000 serum peptides (iProphet iprob value > 0.99%, 15 samples). At the same time, 1900-2100 serum peptides were successfully quantified by DIA (data-independent acquisition) 30 run LC-MS/MS (Liquid Chromatography with tandem mass spectrometry) analysis of 15 clinical samples. It required very low volume of serum sample. Therefore, it is feasible to perform peptidomics analysis from 100 µl of the volume of each sample.

### Essence of the invention - description of the invention

The invention is disclosed in the claims.

Novel method for analysis of serum peptidomics involved the following steps:
According to the invention is characterized in that the methods involves the following steps:
I. Tested serum sample preparation according to which the following steps are performed:
   1.The test serum is incubated with a citrate-phosphate buffer with pH 3.1-3.6 prepared in liquid chromatography-mass spectrometry (LC-MS) grade ultrapure water, purity ≥ 99.9%, wherein the buffer is at a volume of 2900 µl to 100 µl serum sample with regard to the ratio of 29:1 volume per volume (v/v) buffer to serum sample. The incubation step takes 3-5 minutes with mixing at a temperature of 4 to 8°C to dissociate peptides from highly concentrated serum proteins (e.g. albumin, globulin, transferrin, haptoglobin, alpha- 1 -antitrypsin, etc.).
   2. The highly concentrated proteins > 16 to 170 kDa, such as albumin, globulin, transferrin, haptoglobin, alpha- 1 -antitrypsin, etc., masking the low-concentrated < 15 kDa serum peptides from the test serum samples. These highly concentrated proteins interfered with and hindered the identification of low-concentrated < 15 kDa serum peptides. Therefore, the invention enables the single step to separate and purify the serum biomarker peptides from highly concentrated serum proteins.
      Purification of isolated peptides is performed by using hydrophilic-lipophilic balanced columns that lead to the removing of proteins equal or bigger than 15 kDa from peptide enriched and simultaneously purification of peptides using hydrophilic-lipophilic balanced columns to obtain peptides smaller than 15 kDa; Therefore, the invention does not comprise any additional step for highly abundant/concentrated serum protein depletion and precipitation, which ultimately improves the final yield of serum peptides and prevents the losses of the many biomarker peptides during depletion and precipitation steps. Prior to loading the incubated serum samples, the hydrophilic-lipophilic balanced columns are equilibrated with liquid chromatography-mass spectrometry (LC-MS) grade water and activated to LC-MS grade methanol purity ≥ 99.9%/0.2 formic acid volume per volume (v/v).
   3. The next step is the elution of peptides, in which 3-5 washings are performed with LC/MS grade water, purity ≥ 99.9%, to remove the unbound and low-binding peptides from columns. Then the bound serum peptides are eluted from the hydrophilic-lipophilic balanced columns by using water/60-80% methanol/0.1-0.2% formic acid volume per volume (v/v). The peptides with a molecular weight equal to or smaller than 3 to 10 kilodaltons (kDa) are considered interest serum peptides. Therefore, eluted peptides in the aforementioned mixture are applied to 3-10 kilodaltons (kDa) filters, and these filters retain the peptides of more than 3-10 kilodaltons (kDa) in molecular weight. Lastly, all the eluted serum peptides are lyophilized to dryness. The invention does not require any lysis buffer for peptide isolating, further reduction, alkylation step, or enzymatic digestion such as tryptic or chymotrypsin to keep the peptides in a stable environment.
   4. Based on all the above three steps, the invention method is cost-effective, simple to perform, and relatively quick for serum peptidomics sample preparation. Therefore, it facilitates to work of high-throughput peptidomics sample preparation.
   5) Worldwide, the serum is a commonly available sample and routinely used for pathological tests. Hence, serum peptidomics invention methodology is globally applicable for all serum samples from human and non-human species, such as canines, cats, swine, horses, cows, cheetahs, leopards, etc., for serum peptidomics analysis.
II. Analysis of selected serum peptides
   6. The isolated peptides are sequenced based on amino acid sequences with a tandem mass spectrometry tool.
   a) Comprehensive qualitative and quantitative
      The invention employed multi-search engine strategies for creating the spectral library for qualitative and quantitative serum peptidomics, facilitating discrimination between the test conditions. As a result, for the first time, comprehensive qualitative serum peptides are identified. Furthermore, it enables DDA (data-dependent acquisition) to identify a total of 15000-17000 serum peptides (iprophet iprobability value > 0.99%, 15 samples). At the same time, 1900-2100 serum peptides were successfully quantified by DIA (data-independent acquisition) 30 run LC-MS/MS (Liquid Chromatography with tandem mass spectrometry) analysis of 15 clinical samples. Therefore, high throughputs in the context of qualitative and quantitative serum peptidomics are the key to this novel serum peptidomics invention.
   b) Peptide length distribution

The invention facilitates the crucial feature of serum peptidomics by screening a wide range of peptide lengths. These peptides are not generated by trypsin digestion, where the trypsin enzyme cleaves the proteins into every K (lysine) or R (arginine) site. On the contrary, the novel approach allows screening the natural and without any enzymatically digested peptides. Furthermore, the peptide length distribution is wide at 7-30 amino acid residues. Therefore, it can be easily tunable to 1-10 kilo Dalton in a range of natural serum peptides from the stroke samples.

Essence of the invention in details and the study description - approach leading to provision of the invention.

Serum peptidomics samples preparation - novel step is use acidic buffer (pH 3.1 -3.6) for peptides enrichment and another novel single step to deplete/remove the highly abundant protein and purification of serum peptides i.e. removing of proteins equal or bigger than 15 kDa from peptide enriched and simultaneously purification of peptides using hydrophilic-lipophilic balanced columns to obtain peptides smaller than 15 kDa), One step, 2. is sequencing of serum peptides with tandem mass spectrometry, while 3. Qualitative and quantitative data analysis (- the novel combination steps from 3.1 to 3.4 for comprehensive qualitative and quantitative serum peptidomics.

The invention differs from those available approaches; it created a serum peptidomics (based on amino acid sequences) approach. It is purely based on sequencing natural peptides of amino sequences and it includes the following steps.

A brief explanation of methods:
1.Samples; a collection of serum samples from patients/diseases group and respective control groups. 50-100 µl of serum samples from each patient are collected and stored at -80°C immediately until the analysis.
2. Glassware qualities; mass spectrometer is a highly sensitive analytical machine to detect the minute amount of contaminants during the sample preparation. To not compromise the peptides signals, it is necessary to use high grades purity of water and other chemicals.
3. Buffer preparation; unexpected use of low pH buffer for enrichment of peptides and release from complexes with high abundant/carrier proteins. The citrate-phosphate buffer pH 3.1-3.6 was prepared in high-grade water.
4. Incubation; peptidomics samples preparation starts with the incubation by using a glass beaker between the serum samples and an acidic buffer, citrate-phosphate buffer pH 3.1-3.6 for 3-5 minutes (gentle and manual vertexing necessary) in the temperature at 4 to 8°C to dissociate protein-peptide interactions especially the peptides bound to the carrier proteins like albumin. Therefore, is characterized by the unexpected use of low pH buffer for enrichment of peptides and release from complexes with high abundant/carrier proteins.
5. Purification of peptides; single-step purification is performed by using hydrophilic-lipophilic balanced columns. Unexpectedly this column can deplete the highly abundant proteins without losing the peptides and simultaneously, it purifies the peptides from other contaminants in the serum. It means removing of proteins equal or bigger than 15 kDa from peptide enriched and simultaneously purification of peptides using hydrophilic-lipophilic balanced columns to obtain peptides smaller than 15 kDa; Therefore, it allows us to yield more peptides and isolate possible carrier peptides from highly abundant proteins. Prior to loading the incubated serum samples, the columns have been equilibrated with water and activated to high-grade methanol.
6. Elution of peptides; first several washing performed with high-grade water in order to remove the unbound and low-binding peptides to columns. Then the bound serum peptides are eluted from the columns by using water/80% methanol/0.2% formic acid volume per volume (v/v). Further eluted peptides collected have been passed through 3kDalton molecular weight filters. Lastly, peptides of interest are lyophilized to dryness. All dried peptide samples were stored at -80 until the mass spectrometry analysis.
7. Sequencing of peptides with tandem mass spectrometry; the dried serum peptides were further subjected to sequencing with UltiMate^{™} 3000 RSLCnano liquid chromatograph (Thermo Scientific) online coupled with Orbitrap Exploris 480 mass spectrometer (MS) (Thermo Scientific). Indexed retention time (iRT) peptides were added to each sample to control the retention time of serum peptides for downstream quantitative analysis. Analytical peptide separation was performed by a non-linear gradient with mobile phases A and B. Serum peptides eluting from the analytical column were ionized in a nano-electrospray ion source (NSI) connected to a mass spectrometer.
8. DDA (data-dependent acquisition) mass spectrometry measurements; the data-dependent acquisition (DDA) was developed for serum peptidomics. The full scan was followed by the fragmentation of the top 20 most intense precursor ions and the acquisition of their MS/MS spectra. The DDA data acquired at 120000 resolution used the precursor range from m/z 350 - 1650, and only precursor charge states of +1 to +6 were included in the experiment.
9. DIA (data-independent acquisition) mass spectrometry measurements; while maintaining the same LC (Liquid Chromatography) separation parameter, each DIA (data-independent acquisition) cycle was accompanied by the acquisition of 62 precursor windows/scan events. Orbitrap Exploris 480 mass spectrometer operated in positive polarity data-independent mode (DIA) accompanied by a full-scan in profile mode with 60000 resolution. The normalized collision energy and AGC (Automatic Gain Control) optimized for DIA measurement to provide maximum injection time.

### Examples

The novel qualitative and quantitative serum peptidomics methodology applies to human and non-human serum samples. The methodology is not limited to stroke or healthy donor serum samples. To nullify the contingency of the sample batch, fifteen clinical samples; mainly, five samples of each group considered;
1. Serum from a Healthy Donors (n=5),
2. Acute Ischemic Stroke (AIS) (n=5) diagnosed routinely with imaging diagnostics methods computed tomography (CT) or magnetic resonance imaging (MRI),
3. Intracranial Hemorrhage (ICH) Stroke patient samples (n=5) diagnosed routinely with imaging diagnostics methods computed tomography (CT) or magnetic resonance imaging (MRI).

The invention is described more deeply in examples and shown in figures where in figure 1. the method of peptidomics sample preparation is shown, in figure 2. diagram of qualitative analysis comparing identified peptides, in figure 3. checked the performance of developed DIA (data-independent acquisition) method to classical DDA (data-dependent acquisition), in figure 4. venn diagram comparing serum peptide hits between three groups (Healthy volunteers, acute ischemic stroke (AIS) and intracranial hemorrhage (ICH) patient samples),

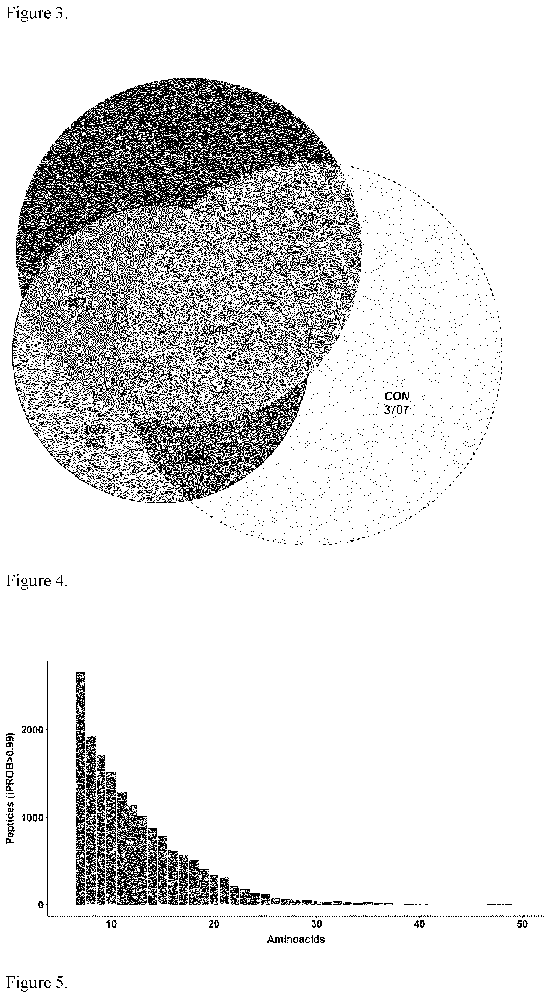

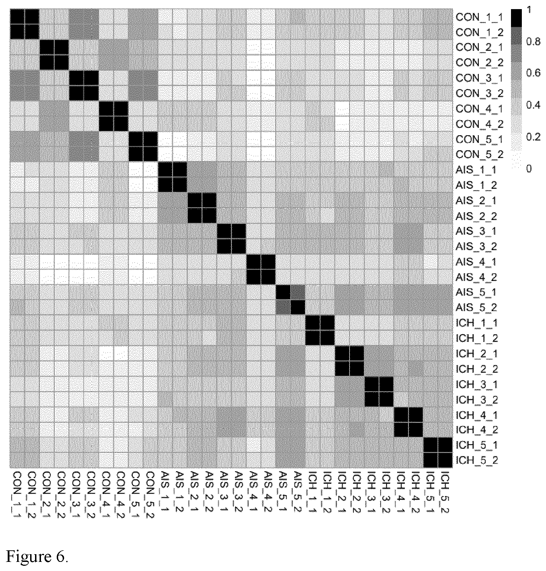

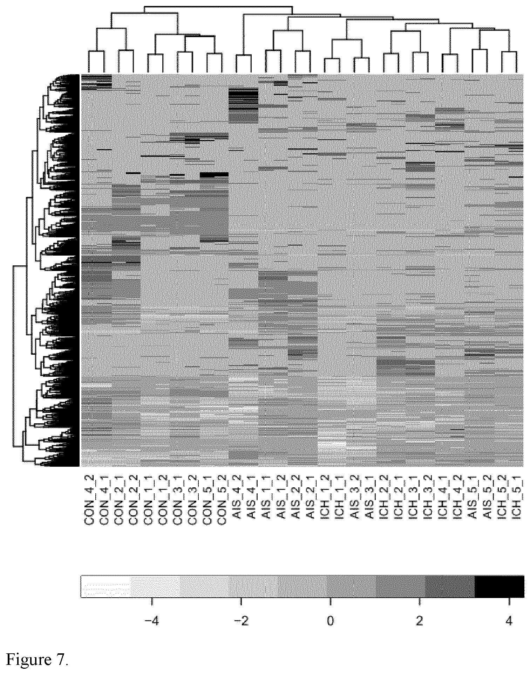

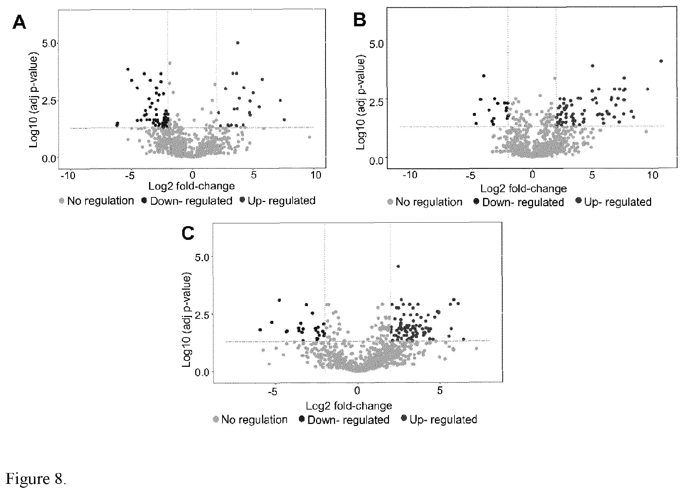

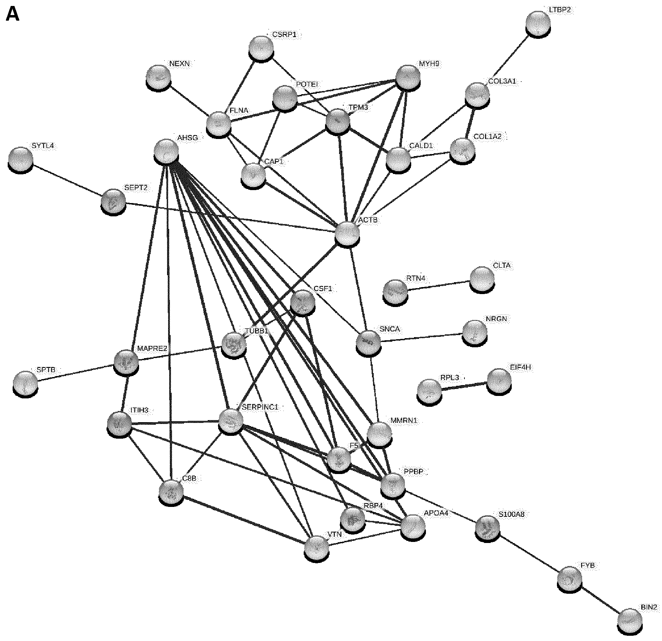

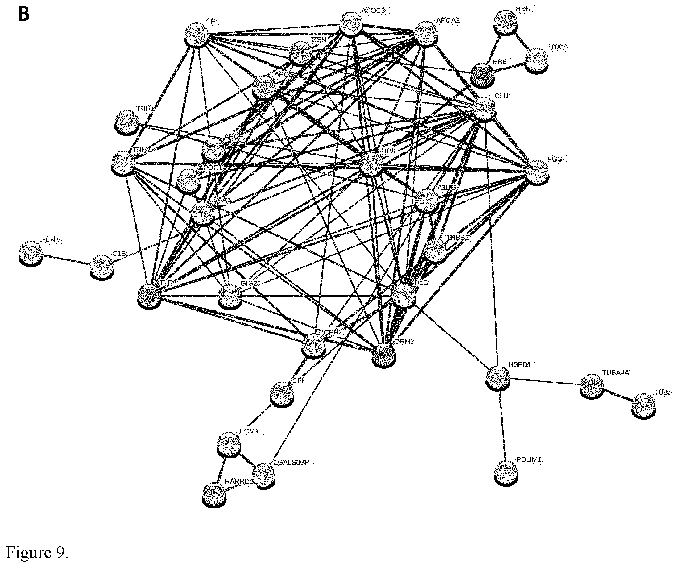

in figure 5. peptide length distribution of serum peptidomics, in figure 6. shows the DDA (data-dependent acquisition)-mass spectrometry (MS) 30 individual run correlation heatmap, in figure 7. shows the peptide heatmap and unsupervised hierarchical clustering between three groups (Healthy donor, acute ischemic stroke (AIS) and intracranial hemorrhage (ICH) patient samples), figure 8. shown the peptide volcano plot of dysregulated serum peptides between three groups (Healthy donor, acute ischemic stroke (AIS) and intracranial hemorrhage (ICH) patient samples), in figure 9. (A, B) shown string analyses for acute ischemic stroke (AIS) and intracranial hemorrhage (ICH) stroke samples determined from their quantitative comparison. The novel and high throughput serum peptidomics approach were employed to prove the concept of serum peptidomics analyses. The samples were collected, serum isolated within one hour, and stored at -80°C until the sample preparation.

### Detailed description of drawings:

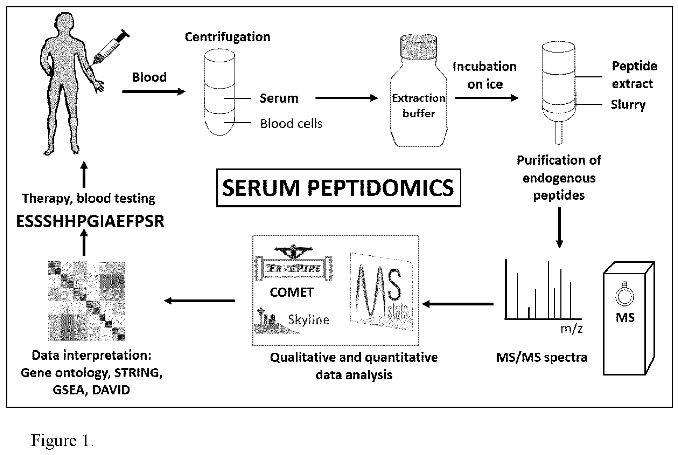

Figure 1. Three steps flowcharts of serum peptidomics sample preparation. The first step is to use acidic buffer incubation for 3 to 5 minutes, allowing the dissociation of protein-peptide interactions and enrichment of peptides. The second key step is purification by oasis columns

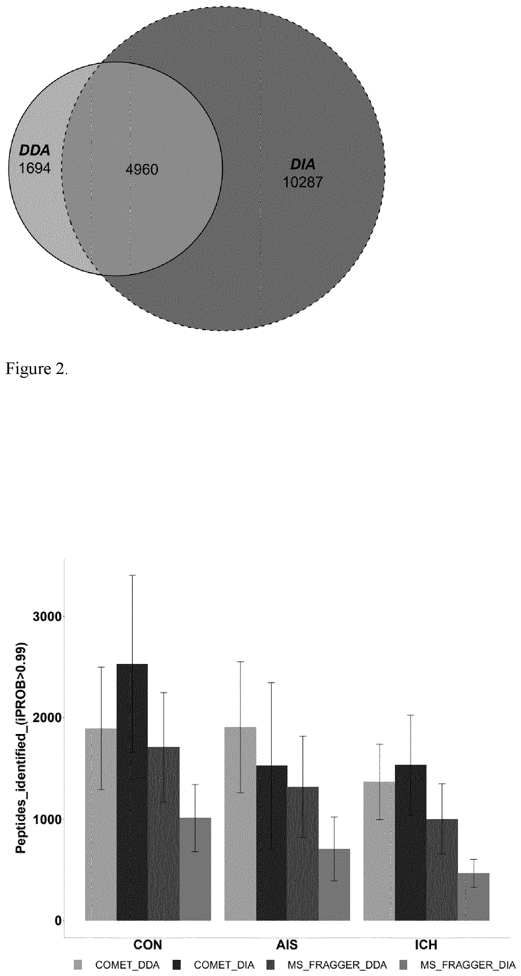

Figure 2. Venn diagram of qualitative analysis comparing identified peptides in DDA (data-dependent acquisition) and DIA (data-independent acquisition) spectrometry-based peptidomics research methods in all peptidome samples.
Figure 3. Performance comparison of developed DIA (data-independent acquisition) method to classical DDA (data-dependent acquisition). (Control/CON, acute ischemic stroke/AIS, intracranial hemorrhage (ICH) stroke. The bar graph reveals the contribution of 4 search approaches to the overall identification.
Figure 4. Venn diagram comparing serum peptide hits between three groups (acute ischemic stroke/AIS, intracranial hemorrhage stroke/ICH, and Control/CON). The relatively small overlap could be again addressed to patient heterogeneity but, in this case, mainly to the different components of patient serum in different patient conditions.
Figure 5. Peptide length distribution of serum peptidomics.
Figure 6. MS (Mass Spectrometry) run correlation heatmap. 30 individual DIA-MS (data-independent acquisition-Mass Spectrometry) runs of serum peptidomes were ordered according to the compared groups (CON/Healthy control [n=5], acute ischemic stroke/AIS stroke [n=5], intracranial hemorrhage stroke/ICH stroke [n=5]). Each patient serum sample was analyzed in a technical duplicate.Squares in heatmap display correlation of log2 transformed peptide intensities.
Figure 7. Peptide heatmap and unsupervised hierarchical clustering of 30 individual stroke patients and healthy donor serum peptidome DIA-MS (data-independent acquisition-Mass Spectrometry) runs. Squares in heatmap display log2 transformed peptide intensities. Color scale extends from white (lowest peptide intensity) up to black (highest peptide intensity). As expected, seen clustering between technical replicates proving excellent performance of LCMS/ MS (Liquid Chromatography with tandem mass spectrometry) system. The heatmap presents similar peptide intensity patterns within compared groups which results in perfect clustering of healthy donor serum peptidomes and same for stroke group. Surprisingly seen a discrimination of clustering between intracranial hemorrhage stroke (ICH) and acute ischemic stroke (AIS) serum peptidomes.
Figure 8. Peptide volcano plot shows (as dark dots) dysregulated serum peptides with significant in comparisons of A. acute ischemic stroke/AIS to Healthy control, B.acute ischemic stroke/AIS to intracranial hemorrhage stroke/ICH, and C.Healthy control to intracranial hemorrhage stroke/ICH.
Figure 9. String analyses of a unique subset of significantly dysregulated protein identifiers in acute ischemic stroke/AIS compared to intracranial hemorrhage (ICH) stroke. Search Tool for the Retrieval of Interacting Genes/Proteins (STRING) analysis determining most common and interacting pathways between selected protein identifiers (adjusted P value (adj.pval) ≤ 0.05, fold-change ≥ 2). (A) STRING nodes characteristic for intracranial hemorrhage stroke (ICH) serum, (B) Search Tool for the Retrieval of Interacting Genes/Proteins (STRING) nodes characteristic for acute ischemic stroke/AIS serum determined from their quantitative comparison.

### Example 1

The method is novel, simple and it has only three steps in sample preparation (Figure
1). Prior to starting any steps of peptidomics method, it is strongly recommended to prepare all the solutions using LC-MS (Liquid Chromatography with mass spectrometry) grade water, solvents, reagents, and ultraclean glass and plasticware use during methods. Moreover, all solutions, and buffers should be prepared immediately before use and disposed of after one week of storage.

### Serum sample collection

The blood samples are collected directly after the stroke patients have been admitted to the hospital by specialized medical staff Additionally, as a control, blood samples from healthy donors samples. All research procedures were performed per the principles of the World Medical Association Declaration of Helsinki. Thus, peptidomics research involved three different groups of patient samples: 1. healthy volunteers (5 donors) 2. patients were suffering from an acute ischemic stroke (AIS) (5 Patients) 3. patients from intracranial hemorrhage (ICH) stroke patients (5 Patients). Furthermore, the clotted sample was centrifuged at 3000 xg for 10 min at 4°C. The serum (supernatant) was stored at -80°C immediately until analysis.

### Buffer preparations

First step was to prepare all the necessary buffers on the same day of peptidomics samples preparation.

1st buffer: citrate-phosphate buffer at pH 3.1 to 3.6 - (0.131 M citric acid/0.066 M Na2HP04, NaCl 150 mM) and adjust the pH 3.1 to 3.6 with 1M NaOH prepared in LC-MS (Liquid Chromatography with mass spectrometry) grade water.

2nd solution: 0.2% formic acid/methanol volume per volume (v/v) was prepared (final proportion of formic acid in methanol was 0.2%).

3rd solution: 0.2% formic acid/water volume per volume (v/v) was prepared (final proportion of formic acid in water was 0.2%).

4th wash buffer: water/5% methanol/0.2% formic acid volume per volume (v/v) was prepared (final proportion of methanol and formic acid in water was 5% and 0.2%, respectively).

5th elution buffer: water/80% methanol/0.2% formic acid volume per volume (v/v) was prepared (final proportion of methanol and formic acid in water was 80% and 0.2%, respectively).

### Peptidome sample preparation

100 µl serum was taken from each sample and incubated with 2.9 ml of citrate-phosphate buffer- at pH 3.1-3 .6 (with optimal results for pH 3.3) for 3-5 minutes (gentle and manual vertexing necessary to dissociate protein-peptide interactions) in the temperature below 4 to 8°C e.g. on the ice. All the above mixtures were mixed e.g. centrifuged at 5000 xg for 5 minutes at 4°C. The supernatant was collected and diluted to 1:1 with 3 ml 0.2% FA in LC-MS (Liquid Chromatography with mass spectrometry) water volume per volume (v/v).

### One step purification of peptides

For the purification of peptides, all the diluted samples (~6ml each) were subjected to the Oasis cartridges (hydrophilic-lipophilic balanced columns, 30 mg; Waters), and all the detailed steps are as follows.
i) Cartridge's condition: cartridge was conditioned with 0.2% formic acid/methanol volume per volume (v/v) (in this solution final proportion of formic acid in methanol was 0.2%), 1 ml/cartridge.
ii) Equilibration of cartridge: the cartridge was equilibrated with 0.2% formic acid/water volume per volume (v/v) (in this solution final proportion of formic acid in water was 0.2%), 1 ml/cartridge.
iii) Sample loading: 6 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1 ml/cartridge.
v) Elution: the bound material (peptides) was eluted with I ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively).

### Peptides preparation for mass spectrometry analysis

The serum peptides sample obtained after peptides purification were spun through 3 kilo Dalton/kDa molecular weight cutoff (MWCO) ultrafiltration devices (AmiconUltra2Centrifugal Filters, Ultracel-3k, 2 mL: Millipore). Briefly, Amicon-Ultracel-3 kilo Dalton/kDa centrifugal filter was rinsed before use (the 3kDa ultrafiltration device was rinsed with 40% methanol with LC-MS (Liquid Chromatography with mass spectrometry) grade water volume per volume (v/v), 1 ml water/column, and centrifuged at 4000 x g, 30 min, 4°C). Next, 3 kDa ultrafiltration device was inserted into the new collecting tube. Lastly, eluted and diluted peptide solution (~2ml) was pipetted into the 3 kDa ultrafiltration device, taking care not to touch the membrane with the pipette tip. Then the 3 kDa ultrafiltration device was span at 4000 x g, 120-135 min, 4°C. The filtrate (peptides) was collected and then lyophilized to dryness. All dried peptide samples were stored at -80 until the mass spectrometry analysis.

(hydrophilic-lipophilic balanced) to eliminate the abundant proteins and simultaneously elute the serum peptides further separated with an ultrafiltration approach using 3 kilo Dalton (3kDa) Molecular weight cutoff (MWCO) filters. The third step is the MS (Mass Spectrometry) measurement and data analysis, resulting serum peptides of each patient were measured three times, one by DDA (data-dependent acquisition) and two DIA-MS (data-independent acquisition)-(Mass Spectrometry) runs.

### Example 2

LC-MS/MS (Liquid Chromatography with tandem mass spectrometry) analysis; the eluted and dried serum peptide samples were resuspended in 30 µl of loading buffer composed of 0.08% trifluoroacetic acid (TFA) in water with 2.5% acetonitrile (ACN). indexed retention time (iRT) peptides (Biognosys) were added according to the manufacturer's guidelines. Then 6 µl of the dissolved sample were injected into UltiMate^{™} 3000 RSLCnano liquid chromatograph (Thermo Scientific) online coupled with Orbitrap Exploris 480 mass spectrometer (MS) (Thermo Scientific,). µ-precolumn C18 trap cartridges (300 µm i.d.) and 5 mm length packed with C18 PepMap 100 sorbent with PepMap 5 µm sorbent (P/N: 160454, Thermo Fisher Scientific) were used to concentrate and desalt serum peptides using a 5 µl/min flow of loading buffer. Peptides were then eluted on 75 µm ID and 150 mm length fused-silica analytical column packed with PepMap 2 µm sorbent (PIN: 164534, Thermo Scientific). Analytical peptide separation was performed by a non-linear increase of a mobile phase B (0.1 % formic acid (FA) in ACN) in a mobile phase A (0.1 % FA in water). A non-linear gradient started at 2.5% B linearly increasing up to 35% B in 80 min, followed by a linear increase up to 60% B in the next 15 min with a flow rate of 300 nl/minute. Serum peptides eluting from the column were ionized in a nano-electrospray ion source (NSI) and were introduced to Exploris 480 (Thermo Scientific).

### DDA (data-dependent acquisition) analysis

The data-dependent acquisition method was prepared in our laboratory. Briefly, Exploris 480 acquired the data in data-dependent peptide mode. The full scan was operated in profile mode with 120000 resolution, scanning the precursor range from m/z 350 Th to m/z 1650 Th. Normalized AGC (Automatic Gain Control) target was set to 300% with 100 msec maximum injection time, and each full scan was followed by fragmentation of the top 20 the most intense precursor ions and acquisition if their MS/MS spectra. The dynamic mass exclusion was set to 20 sec after the first precursor ion fragmentation. Precursor isotopologues were excluded, and mass tolerance was set to 1 0 parts per million (ppm). Minimum precursor ion intensity was set to 3 .0e3 , and only precursor charge states of +1 to +6 were included in the experiment. The precursor isolation window was set to 1.6 Th. Normalized collision energy type with fixed collision energy mode was selected. The collision energy was set to 30% . Orbitrap resolution was set to 60000. Normalized AGC (Automatic Gain Control) target was set to 100% with an automatic setting of maximum injection time, and data type was centroid.

### DIA (data-independent acquisition) analysis

LC (Liquid Chromatography) separation parameters were kept identical to DDA (data-dependent acquisition) acquisition. Orbitrap Exploris 480 mass spectrometer operated in positive polarity data-independent mode (DIA) accompanied by a full-scan in profile mode with 60000 resolution. The full-scan range was set from m/z 350 Th up to m/z 1450 Th, and the normalized AGC (Automatic Gain Control) target was set to 300% with 100 msec maximum injection time. Each DIA (data-independent acquisition) cycle was accompanied by the acquisition of 62 precursor windows/scan events. DIA (data-independent acquisition) precursor range was set from m/z 350 Th up to m/z 1100 Th with 12 Th window width and 1 Th window overlap. Normalized collision energy type with fixed collision energy mode was selected to fragment precursors included within each isolation window. The collision energy was set to 30% and Orbitrap resolution in DIA (data-independent acquisition) mode was set to 30000. Normalized AGC (Automatic Gain Control) target was set to 1000% with automatic setting of maximum injection time while data type was profile.

Initially, compared DIA (data-independent acquisition) and DDA (data-dependent acquisition) based on qualitative results (Venn DIA (data-independent acquisition) vs. DDA (data-dependent acquisition) figure 2) to justify that it has appropriate mass serum peptide isolation protocol and mass spectrometry-based peptidomics research methods. Venn diagram allows us to see the benefit of using any method while combining all patient identifications (IDs) between both approaches. Most of the identifications (IDs) are nicely overlapping; however, there are also unique hits for both DIA (data-independent acquisition) and DDA (data-dependent acquisition).

### Example 3

### Data analysis

First, multi-search engine strategy has been applied for comprehensive qualitative analysis of serum peptidome. DDA (data-dependent acquisition) data were centroided and converted to mzML and mzXML format using MSconvert. Converted MS data were searched using MSFragger 3 .4 embedded in Fragpipe suite and Comet embedded in Trans-Proteomic Pipeline (TPP) 5.2.0 against Homo sapiens SwissProt+TrEMBL reference database concatenated with indexed retention time (iRT) peptide sequence (Biognosys), decoy reverse target sequences and contaminants. The search database was constructed in Fragpipe (v. I5) suite. The following search settings were used for MSFragger: precursor mass tolerance was set to +/-8 parts per million (ppm) and fragment mass tolerance to 10 parts per million (ppm). Enzyme digestion was set to non-specific and peptide length was set from 7 to 45 amino acids. Peptide mass range was set from 200 to 5000 Dalton. Variable modifications were set to: methionine oxidation, protein N-term acetylation, and cysteinylation of cysteine. Data were mass recalibrated, and automatic parameter optimization setting was used to tune fragment mass tolerance. Output file format was set to pep .XML. The following search settings were used for Comet: precursor mass tolerance was set to 8 parts per million (ppm) and fragment mass tolerance to 8 parts per million (ppm). The rest of the settings were identical to MSFragger. The search results (pep.XML files) were processed with PeptideProphet and iProphet as part of the Trans-Proteomic Pipeline (TPP) 5.2.0. Resulting recalculated pep.XML files with peptide iprobabilities (iPROB values) were further processed in Skyline-daily (64-bit, 20.1.9.234).

A spectral library was built from recalculated pep.XML files in Skyline-daily (64-bit, 20.1.9.234). In "Peptide settings" a "Library" tab was selected. An option of "Build" was selected and in the newly popped-up window the "Cut-off score" was set to 0.99 (considers only peptides with false discovery rate (FDR) < 0.01 or 0.99 < iPROB value) and as an indexed retention time standard peptides were selected "Biognosys- 11 (iRT-C18)". All recalculated pep.XML files were loaded and a process of building-up the spectral library was initiated. Next, in the "Digestion" tab in "Background proteome" section the "add" option was selected. In the newly popped up window the background proteome was created from the .FASTA file that was previously used as a reference search library (SwissProt+TrEMBL reference protein database concatenated with indexed retention time peptide sequence (Biognosys), decoy reverse target sequences and contaminants). Next, in the "Library" tab, "Libraries" section the newly built spectral library was selected and the "Explore" button was clicked to explore the library. In the library window an "Associate proteins" option was checked and the "Add all" button was clicked. All peptides including non-unique were added to the document. Skyline automatically generated a retention time calculator based on the indexed retention time peptide retention time values observed in recalculated pep.XML files. The Skyline file was saved on the PC hard disc drive along with newly built spectral library (.blib format) .

Each patient serum sample (a total of 15 patients) were measured using the optimized DDA (data-dependent acquisition)/DIA/SWATH (Sequential Window Acquisition of all Theoretical Mass Spectra) method. Each serum samples were run in one DDA (data-dependent acquisition) and two SWATH (Sequential Window Acquisition of all Theoretical Mass Spectra)/DIA (data-independent acquisition) technical replicates to assure the highest LC-MS/MS (Liquid Chromatography with tandem mass spectrometry) assay quality. DDA (data-dependent acquisition) data were used to generate the spectral library that is not only used for qualitative serum peptidomics but also later necessary to extract quantitative data from DIA/SWATH (Sequential Window Acquisition of all Theoretical Mass Spectra) files. Bar graph diagrams (figure 3) of the identified peptides over (5 DDA (data-dependent acquisition) vs. 5 DIA/one group) represents all serum peptidomes analyzed in three patient groups with our multi-search engine approach described in 'methods' section. Error bars represent a variation of serum peptides no among patients (patient wise, no run wise). Shades of grey color (figure 3) represent number of identified peptides in two data types, namely DDA (data-dependent acquisition) and DIA (data-independent acquisition) each searched with both Comet and MSfragger. Comet search engine performs best searching DIA (data-independent acquisition) data while MSFragger performs perfect DDA (data-dependent acquisition) data search. Performance of search engines highly depends on the search settings for the purpose of the comparison to set the optimal but equal settings for both search engines. The number of peptides identified in stroke patients is significantly lower than that of healthy controls (figure 3). The invention considered only quantified serum peptides in the patients and controls samples in further analysis. It have shown (figure 3) the identification reproducibility. Moreover, the relatively high standard error (STDEV > 17.3) in a number of peptide identifications within the patient group could be addressed to interpatient heterogeneity, which is further visible from venn plot showing the variability among the patient groups presented as a serum peptide identifications (IDs) overlap within patient group s (figure 4). This observation will be present in any dataset and impact the results; however, later show that our approach could deal with this issue.

### Example 4

Another aspect of the invention provides the comprehensive identification of serum peptides from the patient samples. Our DDA (data-dependent acquisition) analysis showed (figure 4) identified a total of 15000 to 17000 serum peptides with high confidence of statistical filtrations (iProphet iprob value > 0.99%, 15 samples acute ischemic stroke/AIS, intracranial hemorrhage stroke/ICH, and Control/CON).

Figure 4 shows a total of ~7000, ~5800, ~4200, serum peptide sequences were identified in Control/CON, acute ischemic stroke/AIS, and intracranial hemorrhage/ICH stroke samples, respectively, with ~4000 (90%) stripped peptides commonly identified. The potential of our novel approach to employ on a large set of clinical sets shows that (figure 4) the total peptides from 3 experimental groups have a high degree of overlap.

### Example 5

The invention highlights other crucial features of serum peptidomics analysis in our DDA (data-dependent acquisition) screens are peptide length distribution and range of molecular weight (figure 5). The peptide length distribution depends on interest; it can easily tune and enrich the short (less than 3 kilo Dalton) and long (less than 10, 30 kilo Dalton) by using 3, 10, 30 kilo Dalton ultrafiltration cutoff membranes, respectively.

In the current analysis, during peptides purification step employed a 3 kilo Dalton (kDa) filter, and hence the distribution was 7-30 amino acid residues, with the 15 amino acid residues as the median value (figure 5). Therefore, the molecular weight range of serum peptides enriched showed that mass distribution ranges between 1-3 kilo Dalton (kDa).

### Example 6

The further application is wholly based on serum peptide quantitation. In a quantitative experiment, first used DDA (data-dependent acquisition) data to generate an appropriate spectral library not losing many important serum peptides at the same time not introducing too many low-confidence serum peptides. Then, used exclusively DIA (data-independent acquisition) data to extract quantitative information about serum peptides included in the spectral library. Furthermore, consider DIA (data-independent acquisition) data as an only choice to properly quantitate serum peptides due to its perfect reproducibility multiplexing. Moreover, as already suggested DIA (data-independent acquisition) data could be used for both identification and precise quantitation of serum peptides. Several methods with distinct window width were developed to accurately quantify stroke-specific markers. The peptides precursor ions distribution was initially screened in the optimized DDA (data-dependent acquisition) method from serum samples. The inventions determined the most populated part of precursor range which covers the most of the peptide masses observed. The rest of the DIA (data-independent acquisition) methods parameters such as normalized AGC (Automatic Gain Control) target, injection time, window overlap were optimized to keep the cycle time up to 3.5 s. Such method cycle In combination with described chromatographic conditions (in method section) will yield at least 10 datapoints pep peak as is a minimum recommendation by FDA (The United States Food and Drug Administration) at the same time keeping sufficient selectivity and sensitivity of the method.

### Quantitative peptidomics data extraction

Continuing with the Skyline file generated in the previous step is highly recommended to set the extraction settings properly. Quantitative values reflecting the peptide quantity in sample (peptide peak areas) were extracted from DIA (data-independent acquisition) data in Skyline-daily (64-bit, 20.1.9.234) based on the peptide-product ion pairs (transitions) listed in the spectral library from previous step. In the "Peptide settings" tab, the "Max. missed cleavages" was set to 0. Next, in the "Filter" sub-tab a maximum peptide length was set from 4 to 200 amino acids. The "Exclude N terminal AAs" option was set to zero. A function of the "Auto-select all matching peptides" was selected. In the "Modifications" sub-tab, no modifications were selected. In the "Library" sub-tab, in the "Libraries" window, a spectral library that was created in the previous step was selected. Other settings in the tab were left default. In "Transition settings" tab, the "Prediction" sub-tab was left default. In the "Filter" sub-tab +1, +2, +3, +5, +6 peptide precursor charges were specified. Ion charges were set to +1 and +2. Ion type was set to y and b. Product ion selection was set as follows: in the "From:" window the "ion 4" and in the "To:" window the "last ion" options were selected. The "Auto select all matching transition" option was selected at the bottom of the sub-tab and in the " Special ions:" menu the "N-terminal to Proline" option was selected. In the "Library" sub-tab, the "Ion match tolerance" window was set to 0.05 m/z and only peptides that have at least 4 product ions were kept in analysis. In addition, if more product ions were available per peptide, then the 6 most intense were picked-up from the filtered product ions. A function of the "From filtered ion charges and types" was chosen. In the "Instrument" sub-tab included product ions from m/z 350 up to m/z 1100. The "Method match tolerance m/z" window was set to 0.055 m/z mass tolerance. mass spectrometry (MS) 1 filtering was set to "none" in the full scan subtab and in tandem mass spectrometry (MS/MS) filtering sub-section, the "Acquisition method" window the "DIA (data-independent acquisition)" option was selected and in the "Product mass analyzer" the "Orbitrap" option was selected. In the "Isolation scheme: " the "Add" option was selected and in the "Edit isolation scheme" pop-up window an option of the "Prespecified isolation windows" was selected. The "Import" isolation windows from DIA (data-independent acquisition).raw file option was then selected and the isolation scheme was read from a DIA (data-independent acquisition) raw file. Back in the "Full-Scan" sub-tab in the "Resolving power:" section the resolving power was set to 30000 at 200 m/z. In the retention time filtering sub-section "Use only scans within 5 minutes of tandem mass spectrometry (MS/MS) identifications (IDs)" was specified. Under the "Refine" sub-tab, the "Document" section of the "Advanced" window the "Min transitions per precursor" option was set to 4. Empty proteins were removed from the document. An equal number of reverse sequence decoys via the "Add Decoys" function in the "Refine" sub-tab was added. In the "Add Decoy Peptides" pop-up window a reverse sequence decoy generation method to create decoy peptides was selected. Following, DIA (data-independent acquisition) ".raw files" were imported. mProphet model to reintegrate peptide product ion peak boundaries in product ion chromatograms was trained as follows: in the "Refine" sub -tab the "Reintegrate" function was selected. In the "Reintegrate" pop-up window in the "Peak scoring model: " the "Add" option was selected. Following, in the "Edit Peak Scoring Model" mProphet model was activated. mProphet model was trained using targets and decoys. The "Score rows" with negative "Weight" and/or "Percentage Contribution" (red highlighted) were removed, and the mProphet model was then re-trained. New mProphet peak scoring model was then selected in the "Edit peak scoring Model" window and applied for reintegration of new peak boundaries. "Export report" function to export a summary of all dependencies required for MSstats 4.0.1. was used to generate quantitative report for downstream analysis. DIA (data-independent acquisition) statistical data analysis in MS stats R-module; Statistical analysis of Skyline extracted quantitative DIA (data-independent acquisition) data was performed in R (version 4.0.0) package MSstats 4.0.1. The protein column was combined with the peptide sequence column to preserve analysis at peptide level, this formatting prevents summing peptide intensities originating from a single protein in MS stats. Extracted peakgroups were reduced by filtering on mProphet q-value < 0.01 cut-off "SkylinetoMSstatsFormat" function was set to keep proteins with one feature and to transform Skyline output to MSstats compatible input. Further, peptide intensities were log2 transformed and quantile normalized. Differential serum peptide quantitation across intracranial hemorrhage stroke (ICH) and acute ischemic stroke (AIS) conditions was performed pairwise via mixed-effect models implemented in MSstats "groupComparison" function. p-values were adjusted using the Benjamini-Hochberg method and output result matrix was exported for downstream analyses. Log2 transformed quantile normalised data matrix was exported to ProBatch 1.8.0 running under R (version 4.0.0) to generate sample correlation heatmaps, dendrograms, analyses. Heatmaps were generated in Heatplus 3.0.0. package, volcano plots in eulerr 6.1.1. package, bar graphs in plyr 1.8.6. and ggplot2 3.3.5.all running under R (version 4.0.0).

With our novel approach, ~1900-2100 serum peptides (The exact method was described i n the "method" section) were successfully quantified by DIA (data-independent acquisition) 30 run LC-MS/MS (Liquid Chromatography with tandem mass spectrometry) analysis of 15 clinical samples. Some peptides are quantitated across all patient groups (acute ischemic stroke /AIS, intracranial hemorrhage stroke/ICH, and Control/CON), showing the wide range (2-135 fold change/FCH) of fold changes across compared patient groups, particularly, signal intensity of the studied peptide of patient groups is at least 2 fold higher (=>2) or at least 2 fold lower (=<-2) than the signal intensity of the control samples. Our result shows a peptides that are completely missing in one of the conditions. This phenomenon provides an infinite changes and p values with zero value. On the comparison with matched control samples identification (qualitative analysis) of at least 15000 serum peptides and quantification (quantitative analysis) of at least 1900 serum peptides can be performed. Followed our quantitative serum peptidomics pipeline to acquire and extract quantitative information about serum peptides from DIA (data-independent acquisition) data. then plotted a MS (Mass Spectrometry) run correlation heat map (figure 6) to compare and correlate 30 individual DIA (data-independent acquisition)-MS (Mass Spectrometry) runs of serum peptidomes. As expected, we see the most correlation between technical replicates (squares in diagonal), proving the excellent performance of the LC-MS/MS (Liquid Chromatography with tandem mass spectrometry) system (R > 0.9). Further, the MS (Mass Spectrometry) run correlation heatmap suggests that healthy donor serum peptidomes are more correlated than stroke patients serum peptidomes (left top square). However, there is a clear difference stratifying between stroke patients and healthy donor serum peptidomes. This discrimination showed that our novel approach fulfills the significant accomplishment of the peptidomics platform. Figure 6, the bottom right part of the heatmap suggests a relatively good correlation (R~0.7) within intracranial hemorrhage stroke (ICH) patient group. On the other hand, acute ischemic stroke (AIS) patients display the highest peptidome heterogeneity. Sample correlation heatmap suggests only slight serum peptidome differences among intracranial hemorrhage stroke (ICH) and acute ischemic stroke (AIS); however, at the same time, it indicates that even minute differences are visible in serum with our peptidomics platform. Overall, a relatively lower correlation coefficient mainly in acute ischemic stroke (AIS) between studied subjects could be addressed to interpatient heterogeneity.

Next, correlated quantitative serum peptidomics signatures with patient groups. Finally, performed unsupervised hierarchical clustering of 30 DIA (data-independent acquisition) LCMS/ MS (Liquid Chromatography with tandem mass spectrometry) runs. Presented peptide heatmap and unsupervised hierarchical clustering (figure 7) show an overview of quantitative peptidomics data analysis among stroke patients and healthy donor serum peptidome DIA-MS runs (data-independent acquisition-Mass Spectrometry).

Surprisingly, similar log2 peptide intensity patterns were observed within compared groups, resulting in a perfect clustering of healthy donor and strokes (AIS, ICH) serum peptidomes. Interestingly, the hierarchical clustering function also reliably discriminates acute ischemic stroke (AIS) from intracranial hemorrhage stroke (ICH) serum peptidomes. Two outlier serum peptidomes clustered differently could result from interpatient heterogeneity mentioned before or different patient clinical history, which was not considered. These data are in excellent agreement with the sample correlation heatmap (figure 6).

### Example 7

The invention next proceeded to provide peptide quantitation among compared patient groups. Significantly dysregulated peptides (adjusted P value (adj.pval) ≤ 0.05, fold-change ≥ 2) were visualized as volcano plots provided in figure 8.

Volcano plots suggest that peptide quantitation provides a list of peptides significantly stratifying between compared patient groups. Hence, it successfully screened significant statistical serum peptides.

### Example 8

To further understand biological significance inferred from serum peptidome quantitative data in stroke patients, employed multi-bioinformatics approaches such as Gene Ontology (GO) enrichment analysis (molecular function, biological process, component analysis), Search Tool for the Retrieval of Interacting Genes/Proteins (STRING), Keyword enrichment (from Uniport) was carried out. Initially, generated interactome maps in Search Tool for the Retrieval of Interacting Genes/Proteins (STRING) to determine characteristic nodes enriched in intracranial hemorrhage stroke/ICH or acute ischemic stroke/ AIS compared respectively. The Search Tool for the Retrieval of Interacting Genes/Proteins (STRING) aims to discriminate specifically between strokes (intracranial hemorrhage stroke/ICH and acute ischemic stroke/AIS). String analyses of significantly dysregulated protein identifiers i n acute ischemic stroke/AIS and intracranial hemorrhage (ICH) stroke. The filtered sub set of unique protein identifiers corresponding to significantly up-regulated peptides (adjusted P value (adj. pval) ≤ 0.05, fold-change ≥ 2) resulting from a comparison of acute ischemic stroke/AIS to intracranial hemorrhage stroke/ICH and intracranial hemorrhage stroke/ICH to acute ischemic stroke/AIS were converted to Uniport protein identifiers. Identifiers common for both up and down-regulated interaction maps were removed, and unique identifiers were subjected to Search Tool for the Retrieval of Interacting Genes/Proteins (STRING) analysis. Analysis shows interesting Search Tool for the Retrieval of Interacting Genes/Proteins (STRING) nodes characteristic for intracranial hemorrhage stroke/ICH serum Figures 9 (A) and (B) for acute ischemic stroke (AIS) serum determined from their quantitative comparison.

This is the kind of biological validation further performed by literature search. More than 2000 quantified peptides were mapped to 200 annotated protein precursors. As shown in figure 9A and B the string analysis reveals the main terms related to (mentioned the exact pathways if any) extracellular exosome, blood microparticle, platelet alpha granule lumen, and extracellular space were most significantly enriched. Thus, the Search Tool for the Retrieval of Interacting Genes/Proteins (STRING) interaction networks provides another potent way of determining key enriched protein nodes from which a predictive role of the peptide could be determined. Hence the novel approach for serum peptidomics sample preparation is comprehensive, qualitative, and quantitative. Mainly, considered serum samples from different strokes (acute ischemic stroke/AIS), intracranial hemorrhage stroke/ICH) and healthy volunteers.Thus, it will revolutionize the early diagnostic field and show a roadmap to the Point of Care Diagnostic (POCD) developments. Ultimately, it will improve the quality of life and be applied to the globe. Therefore, the invention will bring breakthroughs in Point of Care Diagnostic (POCD) platforms and revolutionize the medical field. It is ready to be employed for all kinds (human/non-human) of control/disease serum samples in the future.

### References

[1] R. Richter et al., "Composition of the peptide fraction in human blood plasma: database of circulating human peptides," J. Chromatogr. B. Biomed. Sci. App., vol. 726, no. 1-2, pp. 25-35, Apr. 1999, doi: 10.1016/s0378-4347(99)00012-2.
[2] J. Yang et al., "Serum peptidome profiling in patients with lung cancer," Anat. Rec. Hoboken NJ 2007, vol. 293, no. 12, pp. 2027-2033, Dec. 2010, doi: 10.1002/ar.21267.
[3] Y. Araki et al., "Clinical peptidomic analysis by a one-step direct transfer technology: its potential utility for monitoring of pathophysiological status in female reproductive system disorders," J. Obstet. Gynaecol. Res., vol. 39, no. 10, pp. 1440-1448, Oct. 2013, doi: 10.1111/jog.12140.
[4] L. Yin, Y. Huai, C. Zhao, H. Ding, T. Jiang, and Z. Shi, "Early Second-Trimester Peptidomic Identification of Serum Peptides for Potential Prediction of Gestational Diabetes Mellitus," Cell. Physiol. Biochem., vol. 51, no. 3, pp. 1264-1275, 2018, doi: 10.1159/000495538.
[5] A. A. Abdelati, R. A. Elnemr, N. S. Kandil, F. I. Dwedar, and R. A. Ghazala, "Serum Peptidomic Profile as a Novel Biomarker for Rheumatoid Arthritis," Int. J. Rheumatol., vol. 2020, p. e6069484, Aug. 2020, doi: 10.1155/2020/6069484.
[6] Z. Miao, K. Ding, S. Jin, L. Dai, C. Dai, and X. Li, "Using serum peptidomics to discovery the diagnostic marker for different stage of ulcerative colitis," J. Pharm. Biomed. Anal., vol. 193, p. 113725, Jan. 2021, doi: 10.1016/j.jpba.2020.113725.
[7] H. Ay, "Biomarkers for stroke diagnosis," WO2014121252A1, Aug. 07, 2014 Accessed: Dec. 17, 2022. [Online]. Available: https://patents.google.com/patent/WO2014121252A1/en
[8] T. Garcia-Berrocoso and J. Montaner Vilallonga, "Biomarkers for the Prognosis of Ischemic Stroke," May 01, 2014 Accessed: Dec. 17, 2022. [Online]. Available: https://patentscope.wipo.int/search/en/detail.jsf?docid=WO2014064202
[9] C. Watson, M. Ledwidge, K. Mcdonald, and J. Baugh, "Biomarkers of cardiovascular disease including Irg," WO2011092219A1, Aug. 04, 2011 Accessed: Dec. 17, 2022. [Online]. Available:
   https://patents.google.com/patent/WO2011092219A1/en?oq=PCT%2fEP2011%2f051088
[10] P. Broberg, T. Fehniger, C. Lindberg, and G. MARKO-VARGA, "Peptides as biomarkers of copd," WO2006118522A1, Nov. 09, 2006 Accessed: Dec. 17, 2022. [Online]. Available: https://patents.google.com/patent/WO2006118522A1/en?oq=PCT%2fSE2006%2f000507
[11] J. Klein, M. L. Merchant, R. Ouseph, and R. A. Ward, "Peptide biomarkers of cardiovascular disease," US20140243432A1, Aug. 28, 2014 Accessed: Dec. 17, 2022. [Online]. Available:
   https://patents.google.com/patenUUS20140243432A1/en?oq=US+2014%2f0243432+A1
[12] K. I. VADAKKAN, "Biomarkers for rapid detection of an occurrence of a stroke event," WO2014110674A1, Jul. 24, 2014 Accessed: Dec. 17, 2022. [Online]. Available:
   https://patents.google.com/patent/WO2014110674A1/en?oq=PCT%2fCA2014%2f050023
[13] J. M. Villalonga, A. S. ORIOL, and L. R. PASCUAL, "Biomarkers for stroke prognosis," WO2021009287A1, Jan. 21, 2021 Accessed: Dec. 17, 2022. [Online]. Available:
   https://patents.google.com/patent/WO2021009287A1/en?oq=PCT%2fEP2020%2f070152
[14] T. Linke, S. Doraiswamy, and E. H. Harrison, "Rat plasma proteomics: Effects of abundant protein depletion on proteomic analysis," J. Chromatogr. B, vol. 849, no. 1, pp. 273-281, Apr. 2007, doi: 10.1016/j.jchromb.2006.11.051.
[15] "Albumin depletion of human plasma also removes low abundance proteins including the cytokines - Granger - 2005 - PROTEOMICS - Wiley Online Library." https://analyticalsciencejournals.onlinelibrary.wiley.com/doi/10.1002/pmic.200401331 (accessed Dec. 17, 2022).

## Claims

1. A mass spectrometry method for qualitative and quantitative analysis of peptidome in serum based on amino acid sequencing with a tandem mass spectrometry (MS) tool with signaling intensities measurement of peptides, **characterized in that** the method comprises the following steps:
a) enrichment of peptides in the analyzed serum sample by incubation of a sample with a citrate-phosphate buffer with pH 3.1-3.6 in water with a purity ≥ 99.9%, wherein the buffer and serum volume ratio is 29:1 and the buffer and serum are mixed at a temperature of range 4 to 8°C to dissociate peptides from highly concentrated serum proteins;
b) removing of proteins equal or bigger than 15 kDa from peptide enriched and simultaneously purification of peptides using hydrophilic-lipophilic balanced columns to obtain peptides smaller than 15 kDa;
c) washing the hydrophilic-lipophilic balanced columns with high-grade water purity ≥ 99.9% with 0.2% formic acid (v/v) in order to remove the unbound and low-binding peptides from the columns;
d) eluting of serum peptides bound to the hydrophilic-lipophilic balanced columns by using water / 80% methanol / 0.2% formic acid (v/v);
e) filtrating of the sample through 3 kDa molecular weight filters to collect peptides smaller than 3 kDa;
f) providing lyophilization of the obtained peptides by drying the eluted peptides through lyophilization for tandem mass spectrometry analysis, and then sequencing the isolated peptides based on amino acid sequences with a tandem mass spectrometry followed by intensities signaling measurement in studied serum sample in comparison with the matched control serum samples.

2. The method according to claim 1 wherein based on the comparison with matched control samples identification (qualitative analysis) of at least 15000 serum peptides and quantification (quantitative analysis) of at least 1900 serum peptides is performed.

3. The method according to any of claims 1-2 where peptides finally obtained for the analysis can have various sizes according to the molecular weight filters used to collect peptides, wherein peptides are smaller than 3kDa.

## Patentansprüche

1. Massenspektrometrieverfahren zur qualitativen und quantitativen Analyse des Peptidoms in Serum auf der Grundlage der Aminosäuresequenzierung mit einem Tandem-Massenspektrometrie (MS)-Werkzeug, mit Messung der Signalintensitäten von Peptiden, **dadurch gekennzeichnet, dass** das Verfahren die folgenden Schritte umfasst:
a) Anreicherung von Peptiden in der analysierten Serumprobe durch Inkubation einer Probe mit einem Citrat-Phosphat-Puffer mit einem pH-Wert von 3,1 bis 3,6 in Wasser mit einer Reinheit von ≥ 99,9 %, wobei das Volumenverhältnis von Puffer und Serum 29:1 beträgt, und der Puffer und das Serum bei einer Temperatur im Bereich von 4 bis 8°C gemischt werden, um Peptide aus hochkonzentrierten Serumproteinen zu dissoziieren;
b) Entfernung von Proteinen, die gleich oder größer als 15 kDa sind, aus der Peptidanreicherung und gleichzeitige Reinigung der Peptide mit Hilfe von hydrophil-lipophil ausbalancierten Säulen, um Peptide kleiner als 15 kDa zu erhalten;
c) Waschen der hydrophil-lipophilen-balancierten Säulen mit hochwertigem Wasser einer Reinheit von ≥ 99,9 % mit 0,2 % Ameisensäure (v/v), um die ungebundenen und schwach bindenden Peptide von den Säulen zu entfernen;
d) Elution der an die hydrophil-lipophilen-ausbalancierten Säulen gebundenen Serumpeptide unter Verwendung von Wasser / 80% Methanol / 0,2% Ameisensäure (v/v);
e) Filtrieren der Probe durch 3 kDa-Molekulargewichtsfilter, um Peptide kleiner als 3 kDa zu sammeln;
f) Lyophilisierung der erhaltenen Peptide durch Trocknen der eluierten Peptide durch Lyophilisierung für die Tandem-Massenspektrometrie-Analyse und anschließende Sequenzierung der isolierten Peptide auf der Grundlage von Aminosäuresequenzen mit einer Tandem-Massenspektrometrie, gefolgt von einer Messung der Intensitätssignale in der untersuchten Serumprobe im Vergleich zu den angepassten Kontrollserumproben.

2. Verfahren nach Anspruch 1, wobei auf der Grundlage des Vergleichs mit angepassten Kontrollproben die Identifizierung (qualitative Analyse) von mindestens 15000 Serumpeptiden und die Quantifizierung (quantitative Analyse) von mindestens 1900 Serumpeptiden durchgeführt wird.

3. Verfahren nach einem der Ansprüche 1 bis 2, wobei die schließlich für die Analyse erhaltenen Peptide je nach den zum Sammeln der Peptide verwendeten Molekulargewichtsfiltern verschiedene Größen haben können, und die Peptide kleiner als 3 kDa sind.

## Revendications

1. Une méthode de spectrométrie de masse pour l'analyse qualitative et quantitative du peptidome dans le sérum, basée sur le séquençage des acides aminés à l'aide d'un outil de spectrométrie de masse en tandem (MS), avec mesure des intensités des signaux des peptides, **caractérisée en ce que** la méthode comprend les étapes suivantes:
a) enrichissement des peptides dans l'échantillon de sérum analysé par incubation d'un échantillon avec un tampon citrate-phosphate de pH 3.1-3.6 dans de l'eau d'une pureté ≥ 99,9%, le rapport entre le tampon et le volume de sérum étant de 29:1 et le tampon et le sérum étant mélangés à une température comprise entre 4 et 8°C pour dissocier les peptides des protéines sériques hautement concentrées;
b) élimination des protéines égales ou supérieures à 15 kDa des peptides enrichis et purification simultanée des peptides à l'aide de colonnes équilibrées hydrophiles-lipophiles pour obtenir des peptides inférieurs à 15 kDa;
c) lavage des colonnes équilibrées hydrophiles-lipophiles avec de l'eau de haute pureté ≥ 99,9% avec 0,2% d'acide formique (v/v) afin d'éliminer les peptides non liés et faiblement liés des colonnes;
d) élution des peptides sériques liés aux colonnes équilibrées hydrophiles-lipophiles en utilisant de l'eau / 80% de méthanol / 0,2% d'acide formique (v/v);
e) filtrage de l'échantillon à travers des filtres de poids moléculaire de 3 kDa pour collecter les peptides inférieurs à 3 kDa;
f) lyophilisation des peptides obtenus en séchant les peptides élués par lyophilisation en vue d'une analyse par spectrométrie de masse en tandem, puis séquençage des peptides isolés sur la base des séquences d'acides aminés à l'aide d'une spectrométrie de masse en tandem suivie d'une mesure du signal d'intensité dans l'échantillon de sérum étudié en comparaison avec les échantillons de sérum de contrôle appariés.

2. La méthode selon la revendication 1 dans laquelle, sur la base de la comparaison avec des échantillons de contrôle appariés, l'identification (analyse qualitative) d'au moins 15 000 peptides sériques et la quantification (analyse quantitative) d'au moins 1 900 peptides sériques sont effectuées.

3. La méthode selon l'une des revendications 1 à 2, où les peptides finalement obtenus pour l'analyse peuvent avoir différentes tailles en fonction des filtres de poids moléculaire utilisés pour collecter les peptides, les peptides étant inférieurs à 3kDa.
